(19) Europäisches Patentamt
European Patent Office
Office européen des brevets

(11) **EP 4 207 210 A1**

(12) **EUROPEAN PATENT APPLICATION**

(43) Date of publication:
**05.07.2023 Bulletin 2023/27**

(21) Application number: **21218042.6**

(22) Date of filing: **29.12.2021**

(51) International Patent Classification (IPC):
**G16H 20/17** (2018.01)        **G16H 50/50** (2018.01)

(52) Cooperative Patent Classification (CPC):
**G16H 20/17; G16H 50/50**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Diabeloop**
**38028 Grenoble Cedex 1 (FR)**

(72) Inventors:
• **ROMERO UGALDE, Hector**
**38420 Le Versoud (FR)**
• **DAUDET, Laurent**
**38410 Saint Martin d Uriage (FR)**

(74) Representative: **Fidal Innovation**
**4-6 avenue d'Alsace**
**92400 Courbevoie (FR)**

(54) **COMPUTERIZED METHOD TO ASSESS CONFIDENCE IN A MAIN PREDICTIVE OUTPUT DETERMINED BY A PREDICTIVE MODEL**

(57) A computerized method to assess confidence in at least one main predictive output determined by a temporal predictive model,
the model being adapted to determine a predictive output for a time-based parameter representative of a characteristic of a time-based system for a predetermined future time point based on real time-based data representative of the time-based system,
the main predictive output being a prediction data for a predetermined main future time point, the main predictive output being made at a present time point,
the method comprising the following:
- a prediction computerized module implements the model to determine
- the main predictive output,
- at least one intermediate prediction data at at least one future intermediate time point preceding the main future time point,

- at said at least one future intermediate time point, a comparison computerized module determines a comparison score between
the at least one intermediate prediction data and a real data representative of the time-based system at said intermediate future time point,
- at a confidence time point, a confidence assessment computerized module assigns or denies confidence in the at least one main time-based predictive output according to a confidence assessment method based on the comparison score determined by the comparison module.

[Fig. 2]

**Description**

FIELD OF THE INVENTION

The present invention relates to the field of computerized methods to assess confidence in a main predictive output determined by a deep-learning or machine-learning model.

TECHNOLOGICAL BACKGROUND

More precisely, the invention relates to the assessment of confidence in a predictive output for a time-based parameter representative of a time-based system.

For example, the invention can be used in the field of healthcare, in particular in the field of diabetes, but it is not limited to this use.

In the field of diabetes, it is known to evaluate the concentration of blood glucose, and to inject a quantity of insulin as a function of the measured concentration. Recently, so-called "closed-loop" systems were developed, where a processor is programmed to evaluate a volume rate of insulin to be injected, based on patient data, and to control the injection of insulin based on this evaluation. In addition, the processor can be programmed to evaluate a volume of insulin to be injected in some special circumstances, in particular meals. The quantity can be injected to the patient, subject to the patient's approval. Such systems are also called "semi closed-loop", because of the necessary declaration by the patient of some of these special circumstances.

The measured concentration of blood glucose is often used to predict the future concentration of blood glucose. This prediction is therefore used to calculate the quantity of insulin having to be injected in order to maintain the concentration of blood glucose in acceptable intervals.

An incorrect prediction of the future blood glucose can lead to an irrelevant quantity of insulin calculated leading to a concentration of blood glucose in unacceptable intervals.

That is why, in the example, there is a need to assess confidence in the prediction of the future concentration of blood glucose.

Similarly, for a given time-based system, temporal predictions often lack accuracy in the long term (in the context of the time-based system) and sometimes even in the short term.

That is why there is a need to assess confidence in a temporal prediction.

Following the example of the concentration of blood glucose, some methods have been conceived to address this need. These methods do not only concern the medicine field but have been applied to various industrial fields.

For example, in [GHOSHAL et al. 2021] GHOSHAL, Biraja, TUCKER, Allan, SANGHERA, Bal, et al. "Estimating uncertainty in deep learning for reporting confidence to clinicians in medical image segmentation and diseases detection", Computational Intelligence, 2021, vol. 37, no 2, p. 701-734, authors approximate bayesian neural network using drop weights in the neural network. Drop weights approach consists of suppressing neural networks connections according to a Bernoulli distribution.

In [GHOSHAL et al. 2021] inference should be repeated T times requiring additional computation cost compared with classical deep learning models.

The above method reduces the required hyperparameters and improves computation compared with Bayesian neural networks. However, repeating an inference T times still requires additional computation cost compared with classical deep learning models.

In [PAPERNOT et al. 2018] PAPERNOT, Nicolas et MCDANIEL, Patrick, "Deep k-nearest neighbors: Towards confident, interpretable and robust deep learning", arXiv preprint arXiv:1803.04765, 2018, authors introduced Deep k-Nearest Neighbors (DkNN) which is an hybrid classifier that combines the k-nearest neighbors algorithm with representations of the data learned by each layer of the Deep Neural Network (DNN). A test input is compared to its neighboring training points according to the distance that separates them in the representations. The labels of these neighboring points provide confidence estimates for inputs outside the model's training collector, including for malicious inputs like adversarial examples, and therein provide protections against inputs that are outside the model's understanding.

The computational cost to find all neighbors of a test's input in the training set that is usually large is a disadvantage of this method. Large memory is required since all the output of each layer for each training point should be stored.

Thus, in [PAPERNOT et al. 2018], each time confidence of a given prediction is evaluated, all the neighbors should be found in the training set. If the training set is large, the computation time will be long. Moreover, large memory is required since all the output of each layer for each training point should be stored.

Thus, given the technical problem to assess confidence in a temporal prediction, the present invention aims at solving the disadvantage of the previous methods and to provide a solution usable in a wide variety of technical fields.

SUMMARY OF THE INVENTION

Thus, the invention relates to a computerized method to assess confidence in at least one main predictive output determined by a temporal predictive model,

the model being adapted to determine a predictive output for a time-based parameter representative of a characteristic of a time-based system for a predetermined future time point based on real time-based data representative of the time-based system,
the main predictive output being a prediction data for a predetermined main future time point, the main predictive output being made at a present time point,
the method comprising the following:

- a prediction computerized module implements the model to determine
- the main predictive output,
- at least one intermediate prediction data at at least one future intermediate time point preceding the main future time point,
- at said at least one future intermediate time point, a comparison computerized module determines a comparison score between

the at least one intermediate prediction data and a real data representative of the time-based system at said intermediate future time point,

- at a confidence time point, a confidence assessment computerized module assigns or denies confidence in the at least one main time-based predictive output according to a confidence assessment method based on the comparison score determined by the comparison module.

If the confidence assessment module assigns confidence in the main predictive output, i.e. the main prediction, it can be safely used.
For example, as output of a first computerized module is often an input of a second computerized module, then, if the confidence assessment module assigns confidence in the main prediction, the latter can be used by another module of the general system as an input.
The other module can be another algorithmic module or a module directly responsible for the control of a characteristic of the general system.
According to an example, the characteristic is a physical characteristic. It can be a physiological, biological or a chemical characteristic too. As a contrary, if the confidence is denied, the main predictive output will not be used by any other module of a general system.
Another "use" of the assigned confidence in the main predictive output could be a temporary confidence in the predictive model in general. For example, it is possible to consider that, once confidence in the present main prediction has been assigned, confidence is assigned in the predictive model and therefore confidence in a next main predictive output does not have to be assessed.
As a consequence, the predictive output is immediately used after being calculated. This represents a gain of time.
In an alternative embodiment, the prediction computerized module implements a first model to determine the main predictive output, and the prediction computerized module implements a second and distinct model to determine the at least one intermediate prediction data at at least one future intermediate time point preceding the main future time point,
**[0026]** In other words, in this alternative embodiment, the invention relates to a computerized method to assess confidence in at least one main predictive output determined by a first temporal predictive model,

the first model being adapted to determine a predictive output for a time-based parameter representative of a characteristic of a time-based system for a predetermined future time point based on real time-based data representative of the time-based system,
the main predictive output being a prediction data for a predetermined main future time point,
the main predictive output being made at a present time point,
the method comprising the following:

- a prediction computerized module implements

the first model to determine the main predictive output, and
a second model, distinct from the first model, adapted to determine a predictive output for the time-based parameter representative of the characteristic of the time-based system for a predetermined future time point based on real time-based data representative of the time-based system, to determine at least one intermediate prediction data at at least one future intermediate time point preceding the main future time point,

- at said at least one future intermediate time point, a comparison computerized module determines a comparison score between

the at least one intermediate prediction data and a real data representative of the time-based system at said intermediate future time point,

- at a confidence time point, a confidence assessment computerized module assigns or denies confidence in the at least one main time-based predictive output according to a confidence assessment method based on the comparison score determined by the comparison module.

In one embodiment, the temporal predictive model is a model adapted to determine a predictive output glycemia based on at least a real measured glycemia, the time-based parameter representative of a time-based system being a glycemia measured by a continuous glycemia monitoring system.
In one embodiment, the temporal predictive model is a model adapted to determine any of the predictive output glycemia further based on at least one of the following parameters considered at the present time point when the predictive output is made:

- an insulin quantity delivered parameter,
- a carbohydrate quantity ingested parameter,
- an Insulin Sensitivity Factor parameter, or
- a Carbohydrate-to-Insulin Ratio parameter.

In one embodiment, the prediction module implements the model to determine several intermediate prediction data at several future intermediate time points preceding the main future time point,

- after a predetermined number of predictions, the comparison module determines a comparison score between some intermediate prediction data and some corresponding real point data, and
- the confidence module assigns or denies confidence in the main temporal prediction data output based on the comparison scores determined.

In a preferred embodiment, the comparison module determines a comparison score between all the intermediate prediction data and all the corresponding real point data.
In one embodiment, the prediction module implements the model to determine several intermediate predictions datas at several future intermediate time points preceding the main future time point,

- at each future intermediate time point after each constant time interval, the comparison module determines a comparison score between

each at least one intermediate predictions datas corresponding to the present time point and each corresponding time-based parameter representative of the time-based system, real present time point data representative of the time-based system, and/or intermediate predictions datas corresponding to the present time point made at at least two different timepoints,

- after a predetermined number of predictions, the confidence module assigns or denies confidence in the main temporal prediction data output based on the comparison scores determined.

In one embodiment, the prediction module implements the model to determine several intermediate predictions datas at several future intermediate time points preceding the main future time point, , at each intermediate time point, the confidence computerized module temporary assigns confidence or definitely denies confidence in the main time-based predictive output according to a confidence assessment method based on the comparison score of the intermediate time point determined by the comparison module.
At the last intermediate time point, the confidence computerized module definitely assigns confidence or definitely denies confidence in the main time-based predictive output according to a confidence assessment method based on the comparison score of the last intermediate time point determined by the comparison module .

In one embodiment, the prediction module implements the model to determine several intermediate predictions datas at several future intermediate time points preceding the main future time point, at a time point, the comparison module determines a comparison score for at least two intermediate predictions datas, made at different past time points, each intermediate prediction data being a prediction data for the time point, the module assigns or denies confidence in the main temporal prediction data output based on the comparison score determined.

In one embodiment, the comparison scores determined by the comparison module are aggregated into an aggregated comparison score according to an aggregation method, and the confidence computerized module assesses confidence in the main temporal prediction data output based on the aggregated comparison score.

In one embodiment, and if applicable, the future time points preceding the main future time point are distributed according to one of the following distribution:

- linear distribution over time according to a predetermined constant time interval
- quadratic distribution,
- a distribution wherein each future time point corresponds to a predetermined percentage of the duration until the main future time point.

In one embodiment, the comparison method is one of the followings:

- an absolute error comparison method,
- a slope difference comparison method,
- root-mean-square error comparison method,
- an acceleration or double derivative difference method, or a combination of the previous methods.

In one embodiment, the confidence assessment method is a comparison of the comparison score with a predetermined threshold.

In another example, various thresholds can be set. Each of these thresholds defines a level of confidence in the main prediction: no confidence, low confidence, high confidence, certainty.

Other confidence method can be implemented.

For example, it is possible to create a confidence model implemented on the slope difference comparison, the absolute error comparison and/or the RMSE comparison as entries data which outputs a percentage of the confidence in the main prediction. In this embodiment, the confidence method consists of implementing the confidence model.

A percentage of confidence can be determined based on the comparison score determined or a combination of various combination score determined by different comparison methods.

In one embodiment, the main temporal prediction is a prediction to 30 minutes, 35 minutes, 40 minutes, 45 minutes, 50 minutes, 55 minutes, one hour, 65 minutes, 70 minutes, 75 minutes, 80 minutes, 85 minutes, 90 minutes, and the prediction module implements any combination of the following intermediates temporal predictions : predictions to 5, 10,15, 20, 30, 35, 40 minutes.

In an embodiment, if, at the confidence time point, the confidence assessment computerized module assigns confidence in the main time-based predictive output according to the confidence assessment method based on the comparison score determined by the comparison module, then

- a temporary confidence is assigned to the predictive temporal model, then
- the prediction computerized module implements the model to determine at least one other main predictive output which confidence is already assigned.

In one embodiment, at one other initial time point, the one other initial time point preceding the confidence time point of the main predictive output, the prediction computerized module implements the model to determine

- one other main predictive output, distinct from the main predictive output,
- at least one other intermediate prediction data at at least one other future intermediate time point preceding the one other main future time point,

the one future intermediate time point preceding the one other future intermediate time point,

- at said at least one other future intermediate time point, the comparison computerized module determines one other comparison score between the at least one other intermediate prediction data and another real data representative of the time-based system at said one other intermediate future time point,

- if, a temporary confidence was assigned to the temporal predictive model according to the confidence assigned in the main predictive output, and at one other confidence time point, the confidence assessment computerized module denies confidence in the one other main time-based predictive output according to a confidence assessment method based on the one other comparison score determined by the comparison module,
- then, temporary confidence assigned to the temporal predictive model lapses.

The invention also relates to method for controlling a system wherein:

- a computerized module implements the computerized method according to the invention on at least one main predictive output determined by a temporal predictive model,
- if, at the confidence time point, the confidence assessment computerized module assigns confidence in the main time-based predictive output according to the confidence assessment method based on the comparison score determined by the comparison module, then
- an active system of the system is controlled according to the main predictive output,
- another computerized module is implemented on the main prediction output, and/or
- a temporary confidence is assigned to the temporal predictive model.

The invention also relates to a computerized system to assess confidence in at least one main predictive output determined by a temporal predictive model,

the model being adapted to determine a predictive output for a time-based parameter representative of a characteristic of a time-based system for a predetermined future time point based on real time-based data representative of the time-based system,
the main predictive output being a prediction data for a predetermined main future time point,
the main predictive output being made at a present time point,
the system comprising the following:

- a prediction computerized module adapted to implement the model to determine
- the main predictive output,
- at least one intermediate prediction data at at least one future intermediate time point preceding the main future time point,
- at said at least one future intermediate time point, a comparison computerized module adapted to determine a comparison score between

the at least one intermediate prediction data and a real data representative of the time-based system at said intermediate future time point,

- at a confidence time point, a confidence assessment computerized module adapted to assign or deny confidence in the at least one main time-based predictive output according to a confidence assessment method based on the comparison score determined by the comparison module.

The invention also relates to a computer program for assessing confidence in at least one main predictive output determined by a temporal predictive model, wherein the computer program is adapted, when run on a processor, to cause the processor to implement the method according to the invention.

BRIEF DESCRIPTION OF THE DRAWINGS

Embodiments of the invention will be described below, in relation to the following drawings :

Fig. 1 shows a common predictive temporal model
Fig. 2 shows a predictive temporal model according to one embodiment of the invention.
Fig. 3 and Fig. 4 schematically show a compartmental predictive model.
Fig. 5 schematically shows a recurrent neural network applicable to one embodiment of the invention.
Fig. 6 and 7 show in more details a recurrent neural network applicable to one embodiment of the invention.
Fig. 8 and 9 schematically show a medical system according to one embodiment of the invention.

On the drawings, the same reference signs show the same or similar objects.

DEFINITIONS

Unit of insulin

In the present detailed description, and according to the WHO Expert Committee on Biological Standardization, one international unit of insulin (1 U) is defined as the "biological equivalent" of 34.7 micrograms ($\mu$g) pure crystalline insulin. This unit is the relevant unit for discussing a quantity of insulin to be infused to a patient, and cannot be converted to the International System of Units, because the conversion would depend on which insulin is being used. For the sake of the disclosure of the present invention, it is important that quantities of insulin be expressed in a system meaningful for the invention, the readers and the scientific community.

DETAILED DESCRIPTION

General overview

Time-based system and time-series

A time-based system is defined as a system evolving over time. The evolution over the time can be manifested by several time variations of the real value R of some characteristics of the system. Usually, these characteristics varying over time are called time variables of the system, or hereafter variables of the system.
Moreover, these characteristics can be physical characteristics of the system and, thus, the real value, and in fine the variations over time, of the time variables can be measured, captured and/or acquired by sensors, measurement systems, capture systems and/or acquisition systems adapted to detect and measure these values R over time.
The physical characteristic can be a physiological, biological or a chemical characteristic too. The term "physical" has to be understood in its broader meaning, i.e. which relates to natural phenomenon.
That is to say, at a given time point $T_x$, i.e. at a precise moment in time $T_x$, a physical characteristic of the system has a real value $R_x$ detected by the sensors, the measurement systems, the capture systems and/or the acquisition systems. Then, at another future time point $T_y$ compared to Tx, i.e. $T_y > T_x$, the physical characteristics of the system has a real value $R_y$ detected by the sensors, the measurement systems, the capture systems and/or the acquisition systems.
A concrete example would be the evolution of the pressure (physical characteristic) of a football ball over time. The pressure could have a value $R_x$= 1100 g/cm$^2$ at a time point $T_x$ and, then after a certain duration, the pressure could have a value $R_y$ = 600 g/cm$^2$ at a time point $T_y$.
The pressure example could also apply for meteorology. For example, a depression is an area where the pressure is less than R = 1013.25 hPa at a given time point T by definition. Conversely, anticyclones are defined by pressure above R= 1013.25 hPa at a given time point T. The same area can be at a given time point $T_x$ a depression, i.e. for example with a pressure of value $R_x$ = 980 hPa, and, then after a certain duration, the pressure could have a value $R_y$ = 1030 hPa at a time point $T_y$.
Moreover, these measurement systems are adapted to register (or at least transmit to appropriate register devices) the detected real value R of the time-variable over time.
The registration of the real value R over time of the physical time variables of the system builds a time sequence of the value R over time. In this sequence, the variation of the real value R can be seen, measured and analyzed.
Following the same example of meteorology, pressure over the time is a good insight to predict the weather. Thus, measuring and analyzing the real value R of the pressure over time is important in this field. A better understanding of how to evaluate the pressure in a given location helps predicting the weather in this location.
From a mathematical approach, this sequence of the real value R over the time of the time-based system is a time series.
In mathematics, a time series is a series of data points indexed (or listed or graphed) in time order. Most commonly, a time series is a sequence taken at successive equally spaced points in time. Thus it is a sequence of discrete-time data.
Examples of time series of physical variables are heights of ocean tides over time or blood glucose concentration over time.

Prediction of temporal values

As explained above, a time-series of a variable is a series of data points indexed in time order representing the value R at each time point over time. As time goes from past to future, the real values R from any time-series are present values and immediately after past value. An interesting problem is to try to predict the future value R, i.e. the value of the observed/measured variable at a future time point, knowing the present and/or past values R of the variable. This problem is known as prediction.

In addition, a particular field of prediction is the prediction of time-series of physical characteristics. Those characteristics are physical so, by definition, they follow physical laws and, in particular, determinist physical laws.

As they follow determinist physical laws, thus constant over time, the future is determined by the past and if one wants to predict the future, one has to observe the present and know the physical laws that determine the future based on the past and/or present. This way to address the problem of prediction is sometimes called dynamic prediction.

Thus, to predict the future value R of a time-based system it is necessary to know with precision both the present state of the time-based system and a law, or a model, describing the evolution of the real value R over time.

For example, according to theories of gravity, if an apple fell from a tree, it would be seen to move towards the center of the earth with a specified and constant acceleration.

The present state can be extended to the past states. Moreover, the state of the time-based system can be described by:

I - the time series of the real value R of the physical variable, and

II - a given number of state parameters S describing the time-based system, these parameters being constant over time and not variable over time unlike the values R.

The general law describing the evolution of R can be named the evolution law E or evolution model E of R. The parameters S can also define the evolution model E. Thus, if time is represented in a discrete way by every time-point T of the time-series of the real value R, then if the physical variable is in state $R_x$, i.e. has a value $R_x$, at a present time point $T_x$, then it will be in states $E(R_x)$, $E(E(R_x))$, and so on, at every future time-point.

The values $E(R_x)$, $E(E(R_x))$ are therefore predictions of the variable as they are not real values R but calculated or estimated values based on the real value R, the given number of state parameters S and the evolution model E.

Following the example of the apple fall, the time-based system would be the apple and the variable observed and measured could be its altitude or its speed.

Thus, the evolution model can be a simple model with only the gravity law with no friction forces, but it can be refined by introduction of friction force if necessary. Based on this model, the initial state of the apple such as the form or the weight of the apple, the past values of the speed and/or altitude time-series (at least initial speed and/or initial altitude) and the evolution model E (gravity law), it is possible to predict the future values of the variable speed and/or the variable altitude at any future time point of the fall.

Issues in the prediction of future values of a time-series

In practice, predicting the future values R of the time-series is a difficult and challenging problem for various reasons.

First, as explained above, an accurate prediction requires a good knowledge of the present state of the time-base system. Thus, an accurate prediction requires accurate sensors, measurement systems, capture systems and/or acquisition systems adapted to detect and measure these values R over time.

Moreover, reducing the system to a finite number of state parameters S to describe it can be difficult. Indeed, what are the relevant parameters to select? And, once selected, how to be sure they will not evolve over time, and if they do, how would they evolve? What would be the influence of those evolutions of the parameters S on the evolution model E?

That is why a common question in temporal prediction is the updating over time of the parameters S. This updating is difficult and a poor parameterization of the time-based system would likely lead to poor predictions. Anyway, even if all the parameters S could be known, it would be difficult to verify that all the parameters S are well-defined, because they would be too numerous to be all measured or even unavailable.

Moreover, the conception of the evolution law or the evolution model is also a difficult challenge. Indeed, there is often a plurality of variables (over time) influencing the variable R and building a model based on mathematical laws linking all the variables over time, derived from determinist physics laws, can be very difficult if not impossible.

Finally, dynamic prediction often only provides good results for the short term, i.e. for the near future, even if all state variables have been accurately measured and the knowledge of the evolution law or model is accurate. Accurate knowledge of the dynamics of the system is not always sufficient to guarantee that the real values will be the same as the numerical values or predicted values, because of the sensitivity to initial conditions. The principle is simple: a very small initial difference can have big consequences.

By "near future", it has to be understood as a future near enough for the time-based system to maintain constant the given number of state parameters S describing it.

As a consequence, the temporal prediction at long term for the time-based system defines a future where the given number of state parameters S describing it have changed enough to change the way the time-based system evolves, i.e the evolution model E can have different properties due to the change of the state parameters S. Another way to define the long term is that the time series R observed (or/and the others time series of the variables of the time-based system) has now completely different values.

Algorithm point of view

In complex computerized systems, the output data of a first computerized module is often the input data of a second computerized module. Thus, an inaccuracy in a prediction output realized by a first predictive computerized module can lead to other inaccuracies because the second computerized module will implement computerized operation on an inaccurate input data.

This situation is even worse if the second computerized module is also a predictive module.

For all the previous issues described, there is a need to assess confidence, trust or reliability in predictions realized or calculated.

Solution implemented by the invention

Components of the general system implementing the method

The method can be implemented by a general system adapted to receive measurement data from various sensors, measurement systems, capture systems and/or acquisition systems adapted to detect and measure a physical characteristic.

As it will be explained later, and as described in the general overview, any physical characteristic could be a variable to be predicted.

The general system may include a processing subsystem, a storage subsystem, a user interface, a communication subsystem, a power subsystem. The general system may also include other components (not explicitly shown).

A storage subsystem can store data, in a more or less arranged fashion; and/or other types of information, examples of which are described below. In some embodiments, a storage subsystem can also store one or more computer programs to be executed by a processing subsystem. A user interface can include any combination of input and output devices. A user can operate input devices of a user interface to provide information to the general system and can receive information from the general system via output devices of a user interface.

This general system can also implement a processing subsystem as one or more integrated circuits, e.g., one or more single-core or multi-core microprocessors or microcontrollers. In operation, a processing system can control the operation of the computerized system. In various embodiments, a processing subsystem can execute a variety of computer programs including program code and can maintain multiple concurrently executing programs or processes. At any given time, some or all of the program code to be executed can be resident in the processing subsystem and/or in a storage media such as the storage subsystem.

Finally, the general system has telecommunication modules adapted to receive and transmit data from and to external components.

Modules

Various computerized modules can be implemented by the processor of the general system.

A prediction computerized module is adapted to determine a prediction of a temporal value as defined and explained in the general overview. This prediction is a prediction data.

To determine it, it can be adapted to implement a predictive model.

This predictive model is adapted to determine, calculate or compute a prediction P, i.e. a predicted value P. This prediction is the output of the model from an algorithmic point of view. It can be named the predictive output of the model. This prediction is made about a physical characteristic of a time-based system, varying over time, i.e. a time-based parameter (or characteristic) representative of the time-based system.

Therefore, the prediction is made about a time-based parameter (or characteristic) representative of the time-based system.

In addition, this prediction is made for a predetermined future time point as any prediction as described in the general overview.

By convention, starting from an initial time point $T_0$, a prediction P $\Delta X$ time ahead is a temporal prediction $P_x$ made at the initial time point $T_0$ for a future time point $T_x = T_0 + \Delta X$ where $\Delta X$ is a duration.

The module can also output one or more intermediate predictions. An intermediate prediction is a prediction made at a time point T preceding the time point $T_x$ of the main temporal prediction $P_x$. In other words, $T_0 < T < T_x$.

Moreover, as described in the general overview, the prediction is made based on real present and/or past value of the physical characteristic of the time-based measured by the sensors of the general system. It can also be based on various state parameters S of the time-based system as described in the general overview.

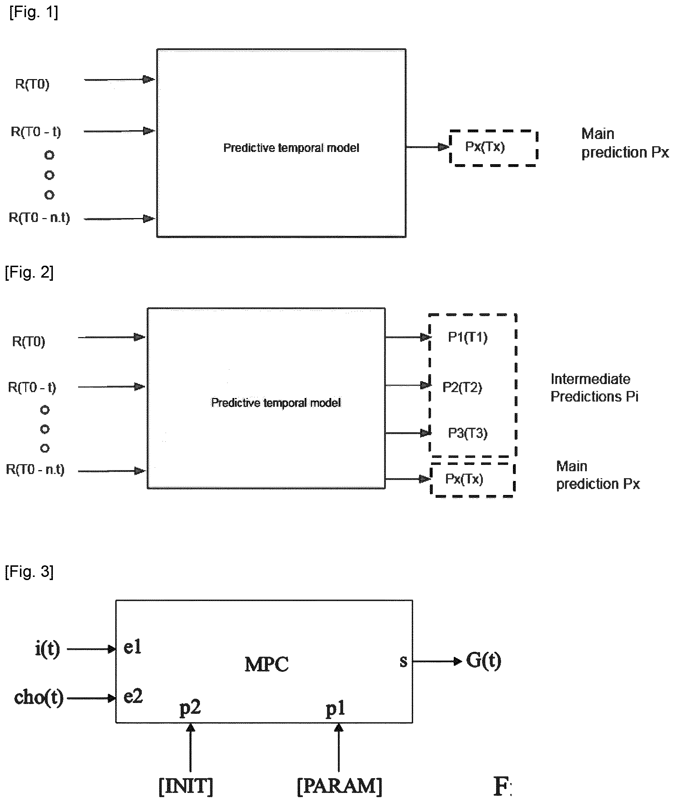

Figure 1 depicted a schema of a predictive temporal model with, as example, three intermediates predictions $P_1$, $P_2$ and $P_3$ made, at a initial time point $T_0$, for the time points $T_1$, $T_2$ and $T_3$, $T_0 < T_1 < T_2 < T_3 < T_x$, and a main prediction $P_x$ made at the same time point $T_0$ for the time point $T_x$.

In addition, the predictive model can be any mathematical model conceived to calculate or compute a future value of the physical characteristic at a future predetermined time-point. As described above, the general state parameters S can define the model.

The entry or input or input data of the model is a real past or current value of the physical characteristic of the time-based and the output or output data is the prediction (named prediction output).

It can be a machine learning or a deep learning model but it is not necessary.

The machine learning or deep learning model outputs the prediction of interest, i.e. the main prediction then used according to any application (see above).

In other words, the output of the model is the main prediction. This main prediction is made for future time points as explained in the general overview.

A comparison computerized module is adapted to compare at least two numerical values or virtual vectors and to determine a comparison score according to a comparison scoring method.

The comparison score can be a comparison score vector if two vectors are compared.

Thus, the inputs of the comparison module are at least two numerical values or virtual vectors and the output is a comparison score (value or vector).

For example, the comparison score can be used to produce indicators, also called mismatch indicators.

In particular, the comparison module is adapted to compare at least two predicted values P, or predictions, i.e. values that have been calculated or computed by the prediction module.

The comparison score method can be chosen according to various alternatives. It can be:

- an absolute error comparison method,

- a slope difference comparison method,

- a root-mean-square error comparison method,

- an acceleration or double derivative difference method.

Each of these comparison score methods can be combined to provide a different comparison score for the confidence assessment method.

All these methods can be weighted.

Other method of mismatch measures between the predicted values and the real values can be used. For example, comparing the acceleration of the predicted signal and the real signal may be used to detect mismatch (acceleration or double derivative method).

The slope difference comparison method is defined as follows.

In mathematics, the slope or gradient of a line is a number that describes both the direction and the steepness of the line.

Slope is calculated by finding the ratio of the "vertical change" to the "horizontal change" between (any) two distinct points on a line or a graph. Sometimes the ratio is expressed as a quotient ("rise over run"), giving the same number for every two distinct points on the same line. A line that is decreasing has a negative "rise". The line may be practical - as set by a road surveyor, or in a diagram that models a road or a roof either as a description or as a plan.

The slope comparison may be defined by the following formulas :

$$slope\_diff = slope\,prediction - slope\,real$$

$$Slope\,real(t) = \left( \frac{\sum_i^L bi \dfrac{R(t) - R(t - T_i)}{T_i}}{L} \right)$$

$$Slope\ prediction(t) = \left( \frac{\sum_i^L \frac{P(t+T_L) - P(t+T_{L-i})}{T_L - T_{L-i}}}{L} \right)$$

but a weighted slope_diff with slope real and slope prediction can also be considered and defined as:

$$Slope\ real(t) = \left( \frac{\sum_i^L bi \frac{R(t) - R(t-T_i)}{T_i}}{\sum_i^L bi} \right)$$

$$Slope\ prediction(t) = \left( \frac{\sum_i^L bi \frac{P(t+T_L) - P(t+T_{L-i})}{T_L - T_{L-i}}}{\sum_i^L bi} \right)$$

According to this comparison method, the slope of the time series of real values $R_i$ and predicted values $P_i$, i.e. predictions, between various time points $T_i$, are aggregated and then compared.

According to the weighted version of this method, the real values $R_i$ and the predicted values $P_i$ are weighted with a weight $b_i$.

This comparison method is particularly relevant to detect drastic changes in the tendency of a temporal variable. For instance, if the real slope indicates that the temporal variable is decreasing and the prediction slope predicts that the temporal variable is increasing then it is almost sure that the main prediction will be wrong.

Moreover, some applications are more about the control of the dynamic of the data time series than the absolute value of the data. This method is therefore relevant for those applications.

The absolute error comparison is defined as follows.

At any time point $T_i$, the real value Ri and the prediction Pi are compared according to the following formula:

$$absolute_{error_{diff(T_i)}} = abs\big(R_i(t) - P_i(t)\big) = abs\big(R(T_0 + T_i) - P(T_0 + T_i)\big)$$

This comparison is made at the last time point $T_L$. In the preferred case, the absolute error comparison is computed by using a weighted mean absolute error that may be computed on the entire intermediate predictions as:

$$absolute_{error_{diff(T_i)}} = \frac{\sum_i^L bi * abs\big(R(T_0 + T_i) - P(T_0 + T_i)\big)}{\sum_i^L bi}$$

This method is relevant in the case of a constant error, between predictions and real values, that may not be detected by slop_diff indicator. As an example, suppose that slope of prediction is the same slope than real values but there is a constant error. This mismatch measure may be an indicator of that constant error. When control decisions are based on the current value or on the current range of the data, minimizing the absolute error can greatly improve the control.

The root-mean-square error comparison is defined as follows.

The root-mean-square deviation (RMSD) or root-mean-square error (RMSE) is a frequently used measure of the differences between values predicted P for or at a time point T by a model or an estimator and the values observed at the same time point T. The RMSD represents the square root of the second sample moment of the differences between the predicted values P and the observed, measured or real values R or the quadratic mean of these differences. The RMSD serves to aggregate the magnitudes of the errors in predictions for various data points into a single measure of predictive power. RMSD is a measure of accuracy, to compare forecasting errors of different models for a particular dataset and not between datasets, as it is scale-dependent.

The following formula describes an example of this type of comparison method:

$$MSE(t) = \sum_{i}^{L} (R_i(t) - P_i(t))^2 = \sum_{i}^{L} (R(T_0 + T_i) - P(T_0 + T_i))^2$$

$$RMSE(t) = \sqrt[2]{\sum_{i}^{L} (R_i(t) - P_i(t))^2} = \sqrt[2]{\sum_{i}^{L} (R(T_0 + T_i) - P(T_0 + T_i))^2}$$

This comparison method is particularly relevant when the collection or measurement of the data implies a constant error of collection or measurement: the slope would not be affected by the constant error of collection.

Other error measurement or deviation measurement between the real values $R_i$ and the intermediate prediction $P_i$ can be used. For example, it is possible to add weights to slope difference, absolute error and/or RMSE.

A confidence assessment computerized module is adapted to assess confidence in a prediction P determined by the prediction module according to a confidence assessment method.

Confidence assessment method

The input of the confidence assessment module is at least a comparison score including at least a prediction P and the output is an assessment of the confidence in this at least one prediction P. The confidence assessment module is adapted to assign or deny confidence in a prediction P according to a confidence assessment method.

The output, the assessment output, can take various formats. It can be a continuous format, i.e. percentage or a score between a lower and an upper limit defining the confidence minimum (no confidence) and the confidence maximum ; or a discrete format, i.e. a binary output for example : 0 for denying confidence and 1 for assigning confidence.

As seen above, indicators can be determined according to the comparison score determined by the comparison module. Then, various thresholds can be set for each indicator based on a comparison score.

A confidence assessment method could be to compare those indicators with the associated threshold: if the indicator is above the given threshold, then the main prediction is not considered accurate and the confidence assessment module does not assign confidence to it.

In another example, various thresholds can be set. Each of these thresholds defines a level of confidence in the main prediction: no confidence, low confidence, high confidence, certainty.

Other confidence method can be implemented.

For example, it is possible to create a confidence model implemented on the slope difference comparison, the absolute error comparison and/or the RMSE comparison as entries data which outputs a percentage of the confidence in the main prediction. In this embodiment, the confidence method consists of implementing the confidence model.

A percentage of confidence can be determined based on the comparison score determined or a combination of various combination score determined by different comparison methods.

Method

We will now describe the method implemented by the computerized general system thanks to the various computerized modules previously described.

As a reminder, the need is to assess confidence in a temporal prediction about a physical characteristic of a time-based system, hereafter named the main prediction $P_x$.

As defined above, at an instant $T_0$, we want to predict the value $P_x$ of a time-based system X time ahead, i.e. the prediction is made at the time point $T_0$ for the future time point $T_x$ with $T_x = T_0 + \Delta X$ where $\Delta X$ is a duration.

This common situation is illustrated in figure 1.

As illustrated in figure 2, the suggestion is to:

- at the time point $T_0$, determine at least an intermediate prediction, i.e.predictions for shorter time horizons, i.e.prediction Y time ahead with $\Delta Y < \Delta X$; then

- evaluate the quality of the prediction $P_y$ Y time ahead at the instant $T_y = T_0 + \Delta Y$ with the real values $R_y$ of the time-based system at $T_y = T_o + \Delta Y$ measured by the sensors of the system,

The evaluation of the quality of $P_y$ gives an insight of the quality of the main prediction $P_x$ since we postulate

that accurate short term prediction made in a past time point tends to indicate that long term prediction made at the same past time point is likely to be accurate too.

In an embodiment, it is also possible that the predictive model (here, the first) implemented to determine the main prediction is not exactly the same as a second predictive model implemented to determine the intermediate prediction. Architecture, training dataset or some parameters such as nodes weight can be different between the first and the second predictive models.

Now we will describe in more details how to implement this suggestion with the system and its modules.

One intermediate time point

At the first step of the method, at the initial time point $T_0$, the prediction computerized module implements the model (also named the predictor) to determine :

- the main predictive output $P_x$,

- at least one intermediate prediction $P_y$ data at at least one future intermediate time point $T_y$ preceding the main future time point $T_x$.

The main predictive (or prediction) output is a prediction data for the predetermined main future time point $T_x$. This main prediction is determined at the initial time point $T_0$.

The intermediate time point $T_y$ can be any moment or time point preceding the time point $T_x$. It can also be called "future time point" since it is a future time point compared to the initial moment $T_o$ when the main predictive $P_x$ output is made.

Then, at said at least one future intermediate time point $T_y$, the comparison computerized module determines a comparison score between the at least one intermediate prediction data $P_y$ and a real data representative $R_y$ of the time-based system at said intermediate time point $T_y$.

As described above, the comparison score can be determined by various methods such as :

- an absolute error comparison method,

- a slope difference comparison method,

- a root-mean-square error comparison method,

- an acceleration or double derivative difference method.

Other methods can be imagined.

The comparison score can be the basis of a mismatch indicator or even be directly it.

Then, a confidence computerized module assesses confidence in the main time based predictive output according to a confidence assessment method based on the comparison score determined by the comparison module.

Thus, the assessment module assigns confidence to the main prediction or, on the contrary, denies confidence to it according to the assessment confidence method described above.

Multiple intermediate time points

As the time-based system is adapted to produce a time series thanks to its sensors, measurement system or capture systems or acquisition system as defined above, the previous method with one intermediate time point $T_y$ can be applied to multiple spaced-apart intermediate time points $T_i$ of the time series produced by the time-based system.

Similarly, the more the intermediate predictions are correct, the more the main prediction is likely to be correct.

For this extended version, the objective is to assess confidence in a predictive output $P_x$ X time ahead or at a time-point $T_x$ which verifies, at the instant $T_o$ when the prediction output is determined, the following relationship : $T_o + \Delta X = T_x$.

Each intermediate time point $T_i$ precedes the time point $T_x$. In other words, $T_o < T_i < T_x$, with i an integer from 1 to any integer n.

Any time point $T_i$ can be selected to be an intermediate point when an intermediate prediction $P_i$ is determined by the prediction computerized module implementing the model.

As an example, three intermediate points $T_1$, $T_2$ and $T_3$ can be used with $T_0 < T_1 < T_2 < T_3 < T_x$.

Predictions $P_1$, $P_2$ and $P_3$ for the time points $T_1$, $T_2$ and $T_3$ will be used, at time instant T3, by the comparison module to compute the comparison score.

It is possible, since at the instant $T_3$, the comparison module has received real values $R_1$, $R_2$ and $R_3$ , thanks to the sensors of the system for $T_1$, $T_2$ and $T_3$, that we compare with predictions $P_1$, $P_2$, and $P_3$ determined at initial instant $T_0$ for instant $T_1$, $T_2$, and $T_3$.

Then, at a time point $T_c$ , the confidence time point, the confidence computerized module assesses confidence in the main time-based predictive output $P_x$ according to a confidence assessment method based on at least a comparison score determined by the comparison module.

If multiple (predetermined number) comparison scores are used to assess confidence by the confidence module, each of the comparison scores used can be weighted.

The time point $T_c$ verifies $T_3 \leq T_c < T_x$. It can be advantageous that confidence is assessed as early as possible. Therefore, according to an advantageous embodiment, $T_c = T_3$, $T_3$ being the last intermediate point as defined.

That is why, if there are more than three intermediate time points, $T_c$ can still be the last intermediate time point.

The example can be generalized to any number of intermediate time points $T_i$.

Distribution of the intermediate time points

As already described, the invention can be generalized to as many intermediate time points as required.

Those points can be distributed in the duration $\Delta X$ according to various temporal distributions.

A simple distribution can be a distribution with a constant time interval between two time points $T_i$. According to this distribution :

$$T_{i+1} = T_i + \Delta t$$

$\Delta t$ being the constant time interval in seconds.

In another example :

$$T_{i+2} - T_{i+1} = 2 \times (T_{i+1} - T_i)$$

It can be advantageous to know as early as possible whether the prediction can be trusted. In this case, setting an intermediate point too close to the point $T_x$ is not relevant.

That is why, whatever the distribution is, the last intermediate point can be set independently, and then the previous intermediate points Ti are set according to the chosen distribution.

For example, the last intermediate point can be set at a time point $T_L$ such as the duration $T_x$ - $T_0$ is twice the duration $T_L$-$T_0$ . In other words, the last intermediate point $T_L$ can be set at the half of the duration X.

Another possibility for the time point distribution is to set each time point $T_i$ at a specific i percentage of the $T_x$ point:

$$T_i - T_0 = \frac{i}{100}(T_x - T_0)$$

Whatever the time distribution is, the first intermediate prediction sets the interval time of the time distribution of the predictions series.

Use of the main prediction $P_x$

If the confidence assessment module assigns confidence in the main prediction, this main prediction can be used. Various applications, or examples of this use, will be described later.

For example, an output of a first computerized module is often an input of a second computerized module, then, if the confidence assessment module assigns confidence in the main prediction $P_x$, the latter can be used by another module of the general system as an input. The other module can be another algorithmic module or a module directly responsible for the control of a physical characteristic of the general system.

On the contrary, if, at any time point (intermediate or last), confidence is denied, the main prediction will not be used by any other module of the general system.

Another "use" of the assigned confidence could be a temporary confidence in the predictive model in general. For example, it is possible to consider that, once confidence in the present main prediction has been assigned, confidence is assigned in the predictive model and therefore confidence in the next main prediction, i.e. the main prediction determined at the time point $T_c$ or the first next one, does not have to be assessed.

This can be generalized to any predetermined number of next main prediction determined at the time point $T_c$. For example, two, three, four, five next main prediction can be made at the time point $T_c$. They will be considered reliable on the basis of the assessment of the present main prediction.

As a consequence, prediction is immediately used after being calculated. This represents a gain of time.

Multiple main predictions $P_x$

The method can be generalized to multiple main predictions $P_x$ made at the same initial time point $T_0$. This embodiment is different from the embodiment where temporary confidence is assigned in the predictive model: in this embodiment, the main predictions are all made at the initial time point $T_0$ whereas in the embodiment where temporal confidence in the predictive model is assigned, the other main prediction is calculated at the confidence time point $T_c$.

APPLICATION

The invention can be used in any technical domain where temporal predictions about time series are present and used. Time series forecasting finds wide application in data analysis. These are just a few of the use cases where the invention might be helpful:

- Meteorology: Forecast of meteorological variables such as temperature, precipitation, wind, etc.

- Economy & Finance: Explanation and forecasting of economic factors, financial indices, exchange rates, etc.

- Marketing: Tracking key business performance indicators, such as sales, revenue / expense, etc.

- Telecommunications: forecasting of call data records, call center workforce management, etc.

- Industry: Control of energy variables, efficient journals, analysis of feelings and behaviors, etc.

- Web: sources of web traffic, clicks and logs, etc.

The applicant reserves any right to disclaim any of these applications if necessary in order to respect patentability criteria.

Diabetes

A particular technical domain for the invention is in the field of diabetes. Now, a specific application of the invention in this field is described.

In this field, it is known to evaluate the concentration of blood glucose, and to inject a quantity of insulin as a function of the measured concentration. Recently, so-called "closed-loop" systems were developed, where a processor is programmed to evaluate a volume rate of insulin to be injected, based on patient data, and to control the injection of insulin based on this evaluation. In addition, the processor can be programmed to evaluate a volume of insulin to be injected in some special circumstances, in particular meals. The quantity can be injected to the patient, subject to the patient's approval. Such systems are also called "semi closed-loop", because of the necessary declaration by the patient of some of these special circumstances.

The measured concentration of blood glucose is often used to predict the future concentration of blood glucose. This prediction is therefore used to calculate the quantity of insulin having to be injected in order to maintain the concentration of blood glucose in acceptable intervals.

Thus, the time-based system is a subject and the physical characteristic varying over time of the time-based system is a concentration of blood glucose over the time. For example, the blood glucose can be measured by a continuous glucose monitoring system.

Moreover, the concentration of blood glucose over time in a subject is considered a time-based system with a temporal inertia of approximately 30 minutes.

Components

This specific application of the invention is applied to the automated infusion of insulin to a Type-I diabetic patient.

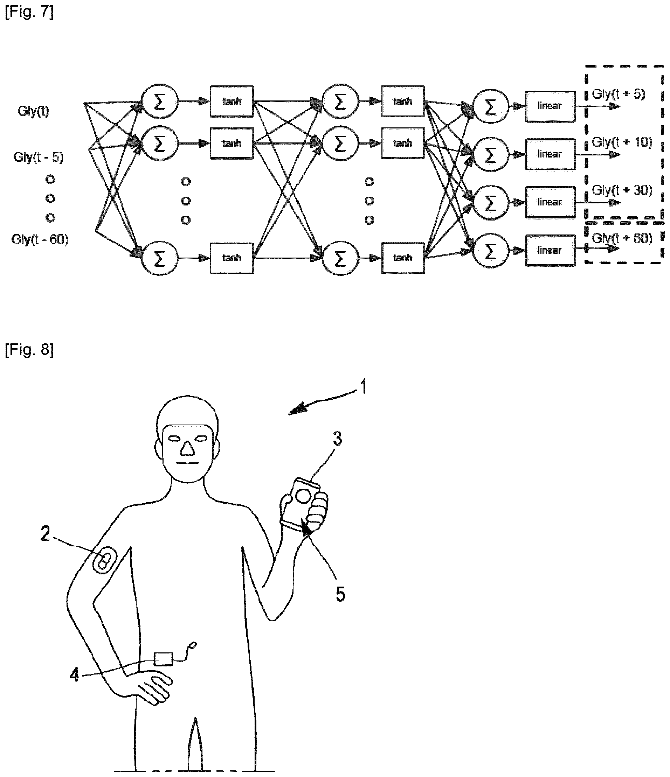

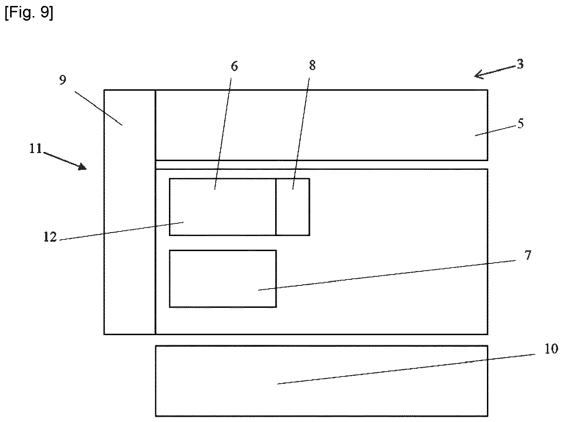

As illustrated in figures 8 and 9 the medical system, considered as the general system, comprises a data acquisition system 2. For example, the data acquisition system comprises a glucose monitoring system adapted to determine a quantity of glucose in the blood of the patient. Various kinds of sensors are applicable to provide this data. One example is a so-called continuous blood glucose monitoring which evaluates a quantity (mass, mol, mass percentage or mol percentage) of glucose in the patient's blood at a high frequency, for example every minute, or even more frequently than that. Various technologies are possible, such as chemical micro-titration or optics. The data acquisition system may comprise additional sensors, such as another glucose monitoring system fixed elsewhere on the patient, or else.

The data acquisition system 2 may also comprise a system to acquire additional sensors' data for instance, accelerometry, temperature, others, that may be indicators of a physiological state of the patient.

The data acquisition system 2 may also comprise a system to acquire additional patient information. This includes for example meal information from the patient. For example, the data acquisition system 2 may comprise an interface allowing the patient to enter meal-related information. Any other information may be input into the medical system 1 through a declarative system.

The medical system comprises a data processing system 3. For example, the data processing system 3 is remote from the blood glucose monitoring system, and is adapted to communicate with it through any suitable means, such as wireless (radiofrequency, such as Bluetooth) or wiredly. However, for example, the above-described declarative interface of the data acquisition system 2 might be provided in the same unit as the data processing system 3. The data processing system 3 is designed to apply the "determination" module 11 on the acquired data, as will be described in more details below, and to determine a set of operating parameters for an active system 4 which, in the present case, comprises a pump.

The medical system 1 comprises an active system 4, which comprises an infusion system. For example, the data processing system 3 is remote from the active system 4, and is adapted to communicate with it through any suitable means, such as wireless (radiofrequency, such as Bluetooth) or wiredly.

Glycemia predictive model

The "determination" module 11 implements a glycemia predictive model or glycemia predictor.

It is a model adapted to determine a predictive output glycemia based on at least a real measured glycemia, hereafter the glycemia prediction model or glycemia predictor. It is a predictive model which predicts a glycemia PG at a predetermined time horizon, i.e.at a predetermined future time point, based on input data. The output is therefore a predicted glycemia PG (which is a prediction data by opposition to the real value i.e.the real data).

The input data includes insulin-on-board (IOB), carbs-on-board (COB), real past or present value of the glycemia RG, insulin, insulin activity. Other pre-defined parameters may be taken into account, such as a parameter related to the patient's sensitivity to insulin.

Thus, the predicted glycemia PG may be determined using a pre-defined evolution function E or predictive model E according to: PG = E (IOB, COB, RG). Any others input data listed above can be used.

Any prediction made by the glycemia predictive model can further be based on one or more of the following parameters: delivered insulin quantity parameter, a consumed carbohydrate quantity parameter, patient-specific parameters such an Insulin Sensitivity Factor, or a Carbohydrate-to-Insulin Ratio parameter.

According to one embodiment, the model E can be a compartmental model comprising, in addition to the input variables i(t) and cho(t) and the output variable PG(t), a plurality of state variables corresponding to the instantaneous values of a plurality of physiological variables of the patient as they evolve over time.

The temporal evolution of the state variables is governed by a system of differential equations comprising a plurality of parameters represented in figure 3 by a vector [PARAM] applied to an input p1 of the MPC block.

The response of the physiological model may also be conditioned by the initial values assigned to the state variables, which is represented in figure 3 by a vector [INIT] applied to an input p2 of the MPC block.

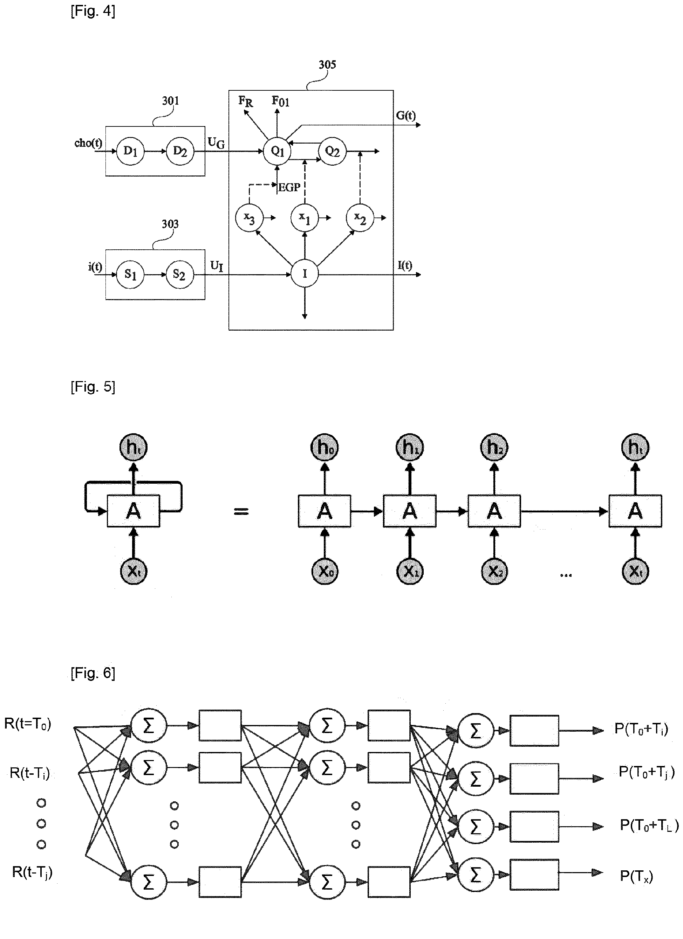

Figure 4 is a diagram which represents in greater detail a non-limiting example of a physiological model implemented in an embodiment of the system of figure 3.

For example, the model can be a Hovorka model as illustrated in figure 3 and described for instance in "Nonlinear model predictive control of glucose concentration in subjects with type 1 diabetes" by Roman Hovorka et al. (Physiol Meas., 2004; 25: 905-920) and in "Partitioning glucose distribution/transport, disposal, and endogenous production during IVGTT" by Roman Hovorka et al. (Physical Endocrinol Metab 282: E992-E1007, 2002).

In this model the predicted glycemia value PG is also named simply G as glycemia.

The physiological model illustrated on figure 4 comprises a first bi-compartmental sub-model 301 describing the effect of glucose intake on the rate of onset of glucose in blood plasma.

Sub-model 301 takes as input a quantity of ingested glucose cho(t), for example in mmol/min, and provides as an output a rate $U_G$ of absorption of glucose in the blood plasma, for example in mmol/min.

In this model, sub-model 301 comprises two state variables $D_1$ and $D_2$ that respectively correspond to glucose masses, for example in mmol, respectively in the first and the second compartment.

The model of figure 4 also comprises a second bi-compartmental sub-model 303 describing the absorption of exogenous insulin delivered to the patient in the blood plasma. Sub-model 303 takes a quantity of exogenous insulin $i(t)$ delivered in the subcutaneous tissue of the patient as an input, for example in mU/min, and provides as an output a rate $U_I$ of absorption of insulin in the plasma, in mU/min.

The sub-model 303 may for instance comprise two state variables $S_1$ and $S_2$, respectively corresponding to on-board insulin which are insulin masses respectively in the first and the second compartments modeling a subcutaneous compartment representative of the subcutaneous tissue of the patient. The instantaneous on-board insulin level of the state variables $S_1$ and $S_2$ may for example be expressed in mmol.

The model of figure 4 may further comprise a third sub-model 305 describing the regulation of glucose by the patient's body. This sub-model 305 takes as inputs the absorption rates $U_G$, $U_I$ of glucose and insulin, and gives as output the blood glucose level $G(t)$, i.e. the concentration of glucose in the plasma, for example in mmol/l.

The sub-model 305 is thus a model of a plasma/body compartment of the patient, i.e. a model of the kinetic and chemical evolution of glucose and insulin in the plasma and the body of the patient.

By "plasma and body of the patient", it is meant the body of the patient with the exception of the subcutaneous tissues.

In this example, the submodel 305 comprises six state variables Q1, Q2, x3, x1, x2, I.

Variables Q1 and Q2 respectively correspond to masses of glucose respectively in the first and the second compartments, for example mmol.

Variables x1, x2, x3 are dimensionless variables respectively representing each of three respective actions of insulin on the kinetics of glucose.

Finally, variable I is an instantaneous plasma insulin level, i.e. corresponds to insulinemia which is a concentration of insulin in the blood plasma. The instantaneous plasma insulin level is for example expressed in mU/l.

The Hovorka model adapted to predict a glycemia G, i.e. to determine a predicted glycemia PG, is governed by the following system of equations:

$$\frac{dS_1}{dt} = i(t) + k_a . S_1(t)$$

$$\frac{dS_2}{dt} = k_a . S_1(t) - k_a . S_2$$

$$\frac{dS_2}{dt} = k_a . S_1(t) - k_a . S_2(t)$$

$$\frac{dI}{dt} = \frac{k_a . S_2(t)}{V_I} - k_e . I(t)$$

$$\frac{dD_1}{dt} = cho(t) - \frac{D_1(t)}{t_{max}}$$

$$\frac{dD_2}{dt} = \frac{D_1(t)}{t_{max}} - \frac{D_2(t)}{t_{max}}$$

$$U_G = \frac{D_2(t)}{t_{max}}$$

$$\frac{dQ_1}{dt} = -\left[ \frac{F_{01}^c}{V_G . G(t)} + x_1(t) \right] . Q_1(t) + k_{12} Q_2(t) - F_R + EGP_0 . [1 - x_3(t)] + U_G(t)$$

$$\frac{dQ_2}{dt} = x_1(t).Q_1(t) - [k_{12} + x_2(t)].Q_2(t)$$

$$\frac{dx_1}{dt} = -k_{b1}.x_1(t) + k_{a1}.I(t)$$

$$\frac{dx_2}{dt} = -k_{b2}.x_2(t) + k_{a2}.I(t)$$

$$\frac{dx_3}{dt} = -k_{b3}.x_3(t) + k_{a3}.I(t)$$

$$PG(t) = G(t) = \frac{Q_1(t)}{V_G}$$

with

$$F_{01}^c = \frac{F_{01}.G(t)}{0.85 \cdot (G(t) + 1..0)}$$

$$F_R = \begin{cases} R(G - 9).V_G & \text{if } G > 9 \\ 0 & \text{otherwise} \end{cases}$$

This system of differential equations comprises fifteen parameters $V_G$, $F_{01}$, $k_{12}$, $F_R$, EGPo, $k_{b1}$, $k_{a1}$, $k_{b2}$, $k_{a2}$, $k_{b3}$, $k_{a3}$, $k_a$, $V_I$, $k_e$ and $t_{max}$ with the following meaning:

$V_G$ corresponds to a volume of distribution of the glucose, for example in liters,
$F_{01}$ corresponds to a non-insulin-dependent glucose transfer rate, for example in mmol/min,
$k_{12}$ corresponds to a transfer rate between the two compartments of sub Model 305, for example in min$^{-1}$,
$k_{a1}$, $k_{a2}$, $k_{a3}$ correspond to an insulin deactivation rate constants, for example in min$^{-1}$,
$F_R$ corresponds to a urinary excretion of glucose, for example in mmol/min,
$EGP_0$ corresponds to an endogenous production of glucose, for example in min$^{-1}$,
$k_{b1}$, $k_{b2}$ and $k_{b3}$ correspond to insulin activation rate constants, for example in min$^{-1}$,
$k_a$ corresponds to a rate of absorption of the insulin injected subcutaneously, for example in min-1,
$V_I$ corresponds to the volume of distribution of the insulin, for example in liters,
$k_e$ corresponds to a plasma elimination rate of insulin, for example in min$^{-1}$,and
$t_{max}$ corresponds to a time to peak glucose absorption ingested by the patient, for example in min.

These fifteen parameters correspond to the vector [PARAM] illustrated on figure Y.
This model further comprises ten state variables $D_1$, $D_2$, $S_1$, $S_2$, $Q_1$, $Q_2$, $X_1$, $X_2$, $X_3$ and I, which are initiated to a vector [INIT] of initial values corresponding to values of these variables at a time step t0 corresponding a beginning of the simulation of the patient's behavior by the model.
The system and method of the invention may also use more simple physiological models than the Hovorka model described above, in particular a compartmental model with a smaller number of state variables and a reduced number of parameters compared to the Hovorka model.
In another embodiment, the model can be a deep-learning model or machine-learning model.
According to this embodiment, the predictive glycemia model E is based on prior data. It does not take into account any pre-defined analytic definition of the physiology of the patient. According to one example, the predictive module E uses a neural network in order to determine the at least one predicted value for the glycaemia of the patient.
The predicted value is determined for a future instant of time, i.e.a future time point T.
The neural network is a machine-learning neural network, which is machine-constructed based on prior patient data or based on a database containing prior data of a group of patients. In an example, the high-level architecture of the neural network may be defined a priori, but the parameters of the neural network may be adapted by the machine-learning process to improve a given situation for a given patient. As another example, both, the high level architecture of the neural network and the parameters of the neural network may be defined a priori for improving a given situation for a given group of patients to which the patient belongs.

In particular, the machine learning model can be a deep learning model. For example, the neural network is a recurrent neural network or a convolutional neural network or other. Upon study, the inventors evaluated that a recurrent neural network,and/or a convolutional neural network are particularly well-suited for prediction of glycaemia based on patient data. In fact, neural networks have shown to be powerful tools for modeling dynamic and complex systems due to their ability to process many inputs, and their ability to approximate any nonlinear function by setting different activation functions. In particular, recurrent neural networks are well-suited to handle temporal data, since they include at least a recurrency node in at least one of their elements (namely neurons or nodes). Recurrency helps the recurrent neural network to understand that the current output of a neuron depends on the input but also on the past output of the systems. In the particular case of blood glucose prediction, the recurrency allows the recurrent neural network to understand that the predicted glucose value does not depends only on the current level of insulin-on-board, or carbohydrate on board and the current value of blood glucose, but also depends on the past values of blood glucose, in other words, recurrent neural networks are able to understand dynamics of a given system.

Figure 5 shows a simplified version of a recurrent neural network suitable to determine a value for a predicted glycaemia based on patient input data. Convolutional neural network is also suitable to determine a value for a predicted glycaemia based on patient input data.

Iterations

In this field, the application is to control the concentration of blood glucose by delivering a bolus of insulin, basal insulin, and/or glucose according to a future prediction of the concentration of blood glucose. For example, the future prediction is a prediction one hour ahead.

Any of the preceding predictive glycemia models can be used by the predictive module.

However, in this detailed embodiment, we assume the glycemia model is a deep-learning or machine-learning model as described above as depicted in figure 7.

In an alternative embodiment, the predictive module can implement a first glycemia model for the main predictions and a second, and different, glycemia model for the intermediate prediction. The two glycemia model can be two machine learning models with different architecture, different nodes weight and/or with the same architecture but trained on different training datasets.

The quantity of insulin to be delivered is determined based, at least, on the main prediction $P_x$, and the insulin pump and/or glucose pump are/is controlled based, at least, on this quantity.

Thus, here for example, the main prediction $PGly_x = PGly(T_0+T_x)$ made at the initial time point $T_0$, as explained above, is the future concentration of blood glucose one hour ahead and the intermediate predictions are predictions of the concentrations of blood glucose at times intermediate between the current time and the time .one hour ahead. Thus, the main prediction is $PGly_{60} = PGly(T_0+60)$.

The main prediction can be made for any of the following future main time point: 40 minutes, 45 minutes, 50 minutes, 55 minutes, 65 minutes, 70 minutes, 75 minutes, 80 minutes.

As described above, the method can be implemented with multiple main predictions made at the initial time $T_0$. The other main prediction can be $PGly_{40} = PGly(T_0+40)$, $PGly_{50} = PGly(T_0+50)$, $PGly_{55} = PGly(T_0+55)$, $PGly_{65} = PGly(T_0+65)$ etc.

Here, for example, the intermediate predictions are set at five, ten and thirty minutes ahead. The intermediate predictions are $PGly_5$, $PGly_{10}$ and $PGly_{30}$.

Other intermediates predictions can be set (and used) at any of the following intermediate time points: 15 minutes, 20 minutes, 25 minutes, 35 minutes etc. As said before, the method can be implemented with at least one intermediate point but it can be two, three, four, five, six, seven or even more.

Thus, the objective is to assess confidence in the future prediction at one hour of the concentration of blood glucose thanks to three intermediate predictions at five, ten and thirty minutes.

In figures 6 and 7, the general overview of the invention according to this specific embodiment is schematized.

As depicted, the model has three intermediate outputs for the three intermediate predictions at five, ten and thirty minutes and one main output for the main prediction at one hour or sixty minutes.

At the first iteration, that is to say at $t=T_0=0$, the glycemia predictor determines the three intermediate predictions $PGly(T_0+5)$, $PGly(T_0+10)$, $PGly(T_0+30)$ and the main prediction $PGly(T_0+60)$ based at least on the real value $RGly(T_0)$.

The first intermediate prediction sets the interval time of the time distribution of the predictions series.

At $t= T_0+5$, the real value $RGly(T_0+5)$ is measured by the continuous glucose monitoring system. Therefore, the comparison module is able to determine, or calculate, a comparison score [PGly($T_0$+5) ; RGly($T_0$+5)] by comparing PGly($T_0$+5) and RGly($T_0$+5) according to any comparison method.

In this specific application of the invention, a preferable comparison method is the slope comparison because a continuous glucose monitoring system can have a broad margin of error but this margin being quite constant over time, the slope is pretty accurate.

Here, the slope comparison method compares the slope between RGly($T_0$) and RGly($T_0$+5) and the slope between RGly($T_0$) and PGly($T_0$+5) (for the very first iteration).

A first comparison score is therefore computed or determined based on any comparison method.

Thus, at this point, the assessment module is already able to assess confidence to the main prediction PGly($T_0$+60) based on the comparison score [PGly($T_0$+5) ; RGly($T_0$+5)] between PGly($T_0$+5) and RGly($T_0$+5). For example, a predetermined threshold for the comparison score or the aggregated comparison score can be set. If the distance (any mathematical distance can be used here) between the threshold and the score is too high, the confidence assessment module can already decide or determine that confidence in the main prediction PGly($T_0$+60) is low. Use of this determination will be described later as the first use.

But, at the same time, i.e.at t'=t+5=$T_0$+5=$T_1$, the glycemia predictor determines three new intermediate predictions PGly($T_1$+5), PGly($T_1$+10), PGly($T_1$+30) and another main prediction PGly($T_1$+60) based at least on the real value RGly($T_1$).

If various comparison scores are computed, any of the comparison scores can be used by the confidence assessment module. It can also be a combination of those scores forming an aggregated score. The combination can include a weighting of the scores, for example a heavier weight for the score of the most recent prediction.

The intermediate prediction PGly($T_1$+5) can be used to assess confidence in the prediction PGly($T_0$+60). Indeed, the time point $T_1$+5 and $T_0$+10 are the same so the intermediates prediction PGly($T_1$+5) and PGly($T_0$+10) are predictions for the same time point but realized at different moments and so based on different real values RGly.

Those two intermediate predictions can be compared in order to assess confidence in the initial main prediction. The prediction PGly($T_1$+5) being a prediction at a shorter horizon than PGly($T_0$+10), this prediction has two advantages over the initial one : it is based on more recent real values and, consequently, the horizon being shorter, the time-based system has less time or possibility to evolve in an unpredictable way. For those reasons, PGly($T_1$+5) is a relevant value to be compared to PGly($T_0$+10) due to the fact that this comparison between two predictions can be done at t = $T_0$+5 = $T_1$ which is sooner than $T_0$+10 = $T_1$+5 when the comparison between PGly($T_0$+10) and RGly($T_0$+10) can be done.

This prediction comparison score [PGly($T_0$+10) ; PGly($T_1$+5)] at t = $T_0$+5 = $T_1$ can be done by the comparison module with the same comparison method except that the real values used are replaced by the previous time-point predictions.

Likewise, at t = $T_0$+5 = $T_1$ the confidence assessment module can use this comparison score between two intermediate predictions for the same time point to assess confidence about the main prediction PGly($T_0$+60) based on the feature that, if the second intermediate prediction for the initial main prediction is consistent with a more recent intermediate prediction for the same time, the other intermediate initial predictions and the main initial prediction are accurate.

Moreover, the confidence assessment module can compare the comparison score [PGly($T_0$+5) ; RGly($T_0$+5)] and the comparison score [PGly($T_0$+10) ; PGly($T_1$+5)].

In particular, a comparison score according to the slope comparison method is particularly relevant because, even if the predictions are not completely accurate in absolute value with the real values, the correlation of the slope of the intermediate predictions at $T_0$ and $T_1$ give a good insight of the accuracy of the main prediction PGly($T_0$+60).

The comparison scores [PGly($T_0$+5) ; RGly($T_0$+5)] and [PGly($T_0$+10) ; PGly($T_1$+5)] are insightful as they compare two time points close to one another but also near the time point $T_0$

In other words, for each intermediate time point T before the last intermediate time point $T_L$ for a main prediction, the confidence assessment module can deny confidence or otherwise assign a potential confidence having to be confirmed at the last time point $T_L$. The idea is to deny confidence as soon as possible if the intermediate predictions are not relevant enough according to the confidence assessment method. The objective is to allow an alternative process as soon as possible in order to have better action over the time-based system and to improve the future prediction which could then be trusted according to the present invention.

In this particular embodiment, the last time point $T_L$ is set at $T_L$ = $T_0$+30. In other words, $T_L$ is set at a horizon being half the horizon of the main prediction. This parameter could be different. However, it is particularly relevant, as it is the last moment when the confidence assessment module can deny confidence in the main prediction.

This potential denial, or the final confidence assessment in the main prediction, is crucial since the use of the main prediction is based on it. Thus, the last time point $T_L$ is preferably quite far from the horizon of the main prediction to allow an alternative process if the confidence in the main prediction has been denied as it will be described later.

Definitive assessment of the confidence

The definitive assessment of the confidence occurs at a predetermined confidence time point $T_c$. In this embodiment, this time point $T_c$ is set to be the last intermediate time point $T_L$.

The comparison module can therefore compare, according to any comparison method, the real value $RGly(T_L = T_0+30)$ with any of the intermediate prediction corresponding to the time point $T_L$, ie, by chronological order, $PGly(T_{L-30}+30)$, $PGly(T_{L-10}+10)$ and $PGly(T_{L-5}+5)$.

The comparison module determines three comparison scores :

- $[RGly(T_L = T_0+30); PGly(T_{L-30}+30)]$,
- $[RGly(T_L = T_0+30); PGly(T_{L-10}+10)]$, and
- $[RGly(T_L = T_0+30); PGly(T_{L-5}+5)]$

With the three comparison scores, potentially combined together into an aggregated comparison score, the confidence assessment module is able to assign or deny confidence in the main prediction $PGly(T_0+60)$ once and for all.

The scores can be weighted in case an aggregated comparison score is determined. For example, the more recent prediction can be weighted more than the oldest one.

In another embodiment, a single comparison score is determined with all the predictions and the corresponding real values: $PGly(T_0+5)$, $PGly(T_0+10)$, $PGly(T_0+30)$ and $RGly(T_0+5)$, $RGly(T_0+10)$, $RGly(T_0+30)$.

In this another embodiment the comparison score is therefore :
$[RGly(T_0+5), RGly(T_0+10), RGly(T_0+30); PGly(T_0+5), PGly(T_0+10), PGly(T_0+30)]$.

The same comparison methods are used on all the intermediate predicted and real values, here the three couple of predicted and real values.

It is possible to determine a single comparison score with only some prediction and their corresponding real values and not strictly all the prediction and their corresponding real values. For example, only a predetermined number of past prediction and their corresponding real values can be taken into consideration by the comparison module to determine the single comparison score.

A preferable method is the comparison of any of the comparison scores for the time point $T_L$ (or an aggregated one) with a predetermined threshold.

This threshold can be determined thanks to a learning stage of the assessment confidence module. This learning consists in denying some predictions considered as not accurate enough to be trusted according to their comparison score compared with the threshold, i.e.to assess confidence in it, and in this case, switch to an alternative process for the management of the future glycemia where the untrusted prediction is not taken into consideration.

That is to say, there are two possibilities: either the main prediction (at a main future time point) is trusted, confidence in it is assessed by the confidence assessment module and this prediction is used for the management of the real glycemia at the main future time point, or the prediction cannot be trusted, confidence in it is denied, and an alternative process for the management of the real glycemia at the main future time point is operated.

By comparing the real glycemia at the main future time point and the predicted glycemia for the same time point, the threshold is adjusted to lower the difference between them until a predetermined accuracy is reached.

By tries and iterations or by using a gradient-based algorithm, an appropriate threshold is computed, learned or determined and can be used by the confidence assessment module.

Same applies for the setting of the time point $T_c$. Here, the $T_L$ point being the $T_c$ point, the setting of the $T_c$ point means defining the last intermediate point $T_L$. This setting is also made tries and iterations or by using a gradient-based algorithm. The selection of the appropriate $T_L$ point is made by comparing the confidence prediction accuracy reached by the method with all the different $T_L$ points possible as described above about the possible intermediate time points possible. It also relies on the main prediction time-point.

Thereby the comparison score for the time point $T_L$ is compared to the threshold, according to an absolute error comparison for example, and confidence in the main prediction is assigned or denied according to this comparison.

Thus, confidence in the main prediction is definitely assigned or is finally denied.

Use of the confidence assessment module decision

Now, at this time point $T_L$ either the confidence has been assigned or denied.

Similarly to the learning stage for the threshold used by the confidence assessment module, there are two possibilities: either the main prediction (at a main future time point) is trusted, confidence in it is assessed by the confidence assessment module and this prediction is used for the management of the real glycemia at the main future time point or the prediction cannot be trusted, confidence in it is denied, and an alternative process for the management of the real glycemia at the main future time point is operated.

From an algorithmic point of view, the decision of the confidence assessment module is responsible for an algorithm node in a computerized glycemia managing system.

As a reminder, the application is to control, with an active system, the concentration of blood glucose by delivering a bolus of insulin, basal insulin, and/or glucose, for example rescue carbs, according to a prediction of the concentration of blood glucose, here the main predicted glycemia (main prediction data).

Thus, for example, a first use of the confidence assessment occurs when the confidence is denied. In this case, the main prediction data, i.e. the main predicted glycemia here, is not used to determine the quantity of insulin or glucose to be injected in order to maintain the glycemia between appropriate limits.

In this first use, the glycemia managing system can implement, for example, an alternative computerized module which comprises pre-defined rules. Notably, the alternative module comprises interpretable rules. For example, it comprises rules set by a doctor to describe a particular medical condition, and to provide a result associated with this particular medical condition. This does not rule out the possibility, for the alternative module, to be regularly updated. An active system of the general system is then controlled on the basis of the output of the alternative module.

Note that this use can occur before the time point $T_L$, at any intermediate time point $T_i$ when the confidence is denied.

This can also occurs when the predictions and the corresponding real values are not available for any reasons. In this case, the determination of the comparison scores cannot be made (at least one) and the reliability of the assessment method can be considered too low. This is particularly the case in the embodiment where a single comparison score is determined with all the predictions and the corresponding real values.

A second use of the confidence assessment occurs when the confidence is assigned to the main prediction, the main predicted glycemia. In this case, the main prediction data, i.e.the main predicted glycemia here, is used to determine the quantity of insulin or glucose to be injected in order to maintain the glycemia between appropriate limits.

An example of this use can be to implement a machine-learning computerized module on the main predicted glycemia (trusted) to determine a set of operational parameters for the active system of the general system. The set of operational parameters are then sent to the active system 4 to control the active system.

A third use of the assessment of the confidence of the main prediction is a temporary confidence assigned in the predictive glycemia model.

According to this embodiment, at least two main predictions have to be considered. The first one, determined at the initial time point $T_0$, which reliability was assigned by the confidence module according to the method as described above at the time point $T_c$, and at least a second main prediction, determined at the time point $T_c$, or few time later

The third use is to assign confidence in the second main prediction, as defined above, on the basis of the confidence assigned in the first main prediction.

This third use can be generalized to any number of other main prediction realized at the time point $T_C$, i.e. a second main prediction, a third main prediction, a fourth main prediction etc. These other main predictions are all made at the confidence time point $T_c$ (or at least at the same moment, few time later the assignment of the reliability for the first main prediction).

The confidence of the second main prediction will not be assessed like the confidence of the first one was but will rather be directly assigned. Thus, the second main prediction can directly be used according to the second use described before.

This third use allows saving time since the confidence is assigned before the time point $T_c$ of the second main prediction timeline. As described earlier, the concentration of blood glucose over the time in a subject is considered a time-based system with a temporal inertia of approximately 30 minutes. Thus, saving 15 minutes, as described in this example, is an important save of time.

Same applies for the generalization at any number of other main predictions, i.e. when multiple main predictions are made at the time point $T_c$. This temporary confidence assigned in the model allows saving time.

Note that, relating to the first main prediction timeline, various other main predictions have been determined during the duration $T_c - T_0$, i.e. before the confidence of the main prediction was assigned (according to the third use), between the first main prediction and the second main prediction as defined above : those main predictions determined are called intermediate main predictions.

The confidence of each of these intermediate main predictions is still assessed according to the method as they are independent of the first main prediction. The confidence of each intermediate main prediction can either be denied at any intermediate time point Ti of their respective timeline or definitely assigned at the confidence time point $T_c$ of their timeline.

If no denying of any confidence occurs, each confidence assigned for each intermediate main prediction can be used according to the third use described (if temporary confidence in the predictive model is assigned) until the confidence of the last intermediate main prediction is used. At this time point, the temporary confidence in the model lapsed and the confidence of the next main prediction is assessed according to the method described above.

If one confidence of an intermediate main prediction is denied at any time point, the temporary confidence in the model lapsed at this time point and the confidence in the next main prediction determined by the predictive model is assessed according to the method described above.

Furthermore, according to the timeline of the first main prediction, assessment of a confidence of one intermediate prediction can occur at the same time point of the assessment of the main first prediction: confidence in the main prediction can be assigned whereas confidence in one intermediate main prediction is denied. In this case, temporary confidence is not assigned and then the confidence of the first main prediction cannot be used according to the third use described above.

## Claims

1. Computerized method to assess confidence in at least one main predictive output determined by a temporal predictive model,

   the model being adapted to determine a predictive output for a time-based parameter representative of a characteristic of a time-based system for a predetermined future time point based on real time-based data representative of the time-based system,
   the main predictive output being a prediction data for a predetermined main future time point,
   the main predictive output being made at a present time point,
   the method comprising the following:

   - a prediction computerized module implements the model to determine

     - the main predictive output,
     - at least one intermediate prediction data at at least one future intermediate time point preceding the main future time point,

   - at said at least one future intermediate time point, a comparison computerized module determines a comparison score between
   the at least one intermediate prediction data and a real data representative of the time-based system at said intermediate future time point,
   - at a confidence time point, a confidence assessment computerized module assigns or denies confidence in the at least one main time-based predictive output according to a confidence assessment method based on the comparison score determined by the comparison module.

2. Method according to the preceding claim wherein:

   - the temporal predictive model is a model adapted to determine a predictive output glycemia based on at least a real measured glycemia,
   - the time-based parameter representative of a time-based system is a glycemia measured by a continuous glycemia monitoring system.

3. Method according to the preceding claim wherein the temporal predictive I is a model adapted to determine any of the predictive output glycemia further based on at least one of the following parameters considered at the present time point when the predictive output is made:

   - an insulin quantity delivered parameter,
   - a carbohydrate quantity delivered parameter,
   - an Insulin Sensitivity Factor parameter, or
   - a Carbohydrate-to-Insulin Ratio parameter.

4. Method according to any of the preceding claims in which:

   - the prediction module implements the model to determine several intermediate prediction data at several future intermediate time points preceding the main future time point,
   - after a predetermined number of predictions, the comparison module determines a comparison score between some intermediate prediction data and some corresponding real point data,
   - the confidence module assigns or denies confidence in the main temporal prediction data output based on the comparison score determined.

5. Method according to any of any of the claims 1 to 4 in which,

- the prediction module implements the model to determine several intermediate predictions datas at several future intermediate time points preceding the main future time point,
- after each constant time interval, the comparison module determines a comparison score between

at least one intermediate predictions datas corresponding to the present time point and a corresponding real present time point data representative of the time-based system, and/or
intermediate predictions datas corresponding to the present time point made at at least two different time points,

- after a predetermined number of predictions, the confidence module assigns or denies confidence in the main temporal prediction data output based on the comparison scores determined.

6. Method according to any of the preceding claims according to which

- the prediction module implements the model to determine several intermediate predictions datas at several future intermediate time points preceding the main future time point,
- at each intermediate time point, the confidence computerized module temporarily assigns confidence or definitely denies confidence in the main time-based predictive output according to a confidence assessment method based on the comparison score of the intermediate time point determined by the comparison module.
- at the last intermediate time point, the confidence computerized module definitely assigns confidence or definitely denies confidence in the main time-based predictive output according to a confidence assessment method based on the comparison score of the last intermediate time point determined by the comparison module .

7. Method according to any of the preceding claims according to which:

- the prediction module implements the model to determine several intermediate predictions datas at several future intermediate time points preceding the main future time point,
- at a time point, the comparison module determines a comparison score for at least two intermediate predictions datas, made at different past time points,

each intermediate prediction data being a prediction data for the time point,

- the module assigns or denies confidence in the main temporal prediction data output based on the comparison score determined.

8. Method according to any of the claims 5 to 7 according to which:

- the comparison scores determined by the comparison module are aggregated into an aggregated comparison score according to an aggregation method, and
- the confidence computerized module assesses confidence in the main temporal prediction data output based on the aggregated comparison score.

9. Method according to any of the claims 4 to 8 according to which the future time points preceding the main future time point are distributed according to one of the following distribution:

- linear distribution over time according to a predetermined constant time interval
- quadratic distribution,
- a distribution wherein each future time point corresponds to a predetermined percentage of the duration until the main future time point.

10. Method according to any of the preceding claims according to which the comparison method is one of the followings:

- an absolute error comparison method,
- a slope difference comparison method,

- a root-mean-square error comparison method,
- an acceleration or double derivative difference method, or
- a combination of the previous methods.

**11.** Method according to any preceding claims wherein,

- if, at the confidence time point, the confidence assessment computerized module assigns confidence in the main time-based predictive output according to the confidence assessment method based on the comparison score determined by the comparison module, then
- a temporary confidence is assigned to the predictive temporal model, then
- the prediction computerized module implements the model to determine at least one other main predictive output which confidence is already assigned.

**12.** Method according to any preceding claims wherein the method further comprises the following:

- at one other initial time point, the one other initial time point preceding the confidence time point of the main predictive output, the prediction computerized module implements the model to determine

    - one other main predictive output, distinct from the main predictive output,
    - at least one other intermediate prediction data at at least one other future intermediate time point preceding the one other main future time point,

the one future intermediate time point preceding the one other future intermediate time point,
- at said at least one other future intermediate time point, the comparison computerized module determines one other comparison score between
the at least one other intermediate prediction data and another real data representative of the time-based system at said one other intermediate future time point,
- if,

    a temporary confidence was assigned to the temporal predictive model according to the confidence assigned in the main predictive output, and
    at one other confidence time point, the confidence assessment computerized module denies confidence in the one other main time-based predictive output according to a confidence assessment method based on the one other comparison score determined by the comparison module,

- then,
temporary confidence assigned to the temporal predictive model lapses.

**13.** Method for controlling a system wherein:

- a computerized module implements the computerized method according to any claims 1 to 12 on at least one main predictive output determined by a temporal predictive model,
- if, at the confidence time point, the confidence assessment computerized module assigns confidence in the main time-based predictive output according to the confidence assessment method based on the comparison score determined by the comparison module, then

    - an active system of the system is controlled according to the main predictive output,
    - another computerized module is implemented on the main prediction output, and/or
    - a temporary confidence is assigned to the temporal predictive model.

**14.** Computerized system to assess confidence in at least one main predictive output determined by a temporal predictive model,

the model being adapted to determine a predictive output for a time-based parameter representative of a characteristic of a time-based system for a predetermined future time point based on real time-based data representative of the time-based system,
the main predictive output being a prediction data for a predetermined main future time point,
the main predictive output being made at a present time point,

the system comprising the following:

- a prediction computerized module adapted to implement the model to determine

    - the main predictive output,
    - at least one intermediate prediction data at at least one future intermediate time point preceding the main future time point,

- at said at least one future intermediate time point, a comparison computerized module adapted to determine a comparison score between
the at least one intermediate prediction data and a real data representative of the time-based system at said intermediate future time point,
- at a confidence time point, a confidence assessment computerized module adapted to assign or deny confidence in the at least one main time-based predictive output according to a confidence assessment method based on the comparison score determined by the comparison module.

15. A computer program for assessing confidence in at least one main predictive output determined by a temporal predictive model, wherein the computer program is adapted, when run on a processor, to cause the processor to implement the method of any of the claims 1 to 13.

[Fig. 1]

R(T0) →
R(T0 - t) →
○
○
○
R(T0 - n.t) →

Predictive temporal model

→ Px(Tx)

Main
prediction Px

[Fig. 2]

R(T0) →
R(T0 - t) →
○
○
○
R(T0 - n.t) →

Predictive temporal model

→ P1(T1)
→ P2(T2)
→ P3(T3)

Intermediate
Predictions Pi

→ Px(Tx)

Main
prediction Px

[Fig. 3]

i(t) → e1
cho(t) → e2

MPC

s → G(t)

p2          p1

[INIT]      [PARAM]

F:

[Fig. 4]

[Fig. 5]

[Fig. 6]

[Fig. 7]

[Fig. 8]

[Fig. 9]

Europäisches
Patentamt
European
Patent Office
Office européen
des brevets

# EUROPEAN SEARCH REPORT

**Application Number**

EP 21 21 8042

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CA 3 055 770 A1 (MEDTRONIC MINIMED INC [US]) 27 September 2018 (2018-09-27) * paragraphs [0125], [0007], [0131], [0135], [0263] * ----- | 1-15 | INV. G16H20/17 G16H50/50 |
| X | CN 101 689 224 A (HOFFMANN LA ROCHE) 31 March 2010 (2010-03-31) * page 69, paragraph 1 * ----- | 1-15 | |
| A | US 2020/282141 A1 (ROUSSON SYLVAIN [FR] ET AL) 10 September 2020 (2020-09-10) * paragraphs [0021], [0147], [0019] * ----- | 1-15 | |
| A | JP 2005 326943 A (OKUMURA ATARU) 24 November 2005 (2005-11-24) * paragraphs [0011], [0012], [0187], [0110] * ----- | 1-15 | |
| A | CN 111 542 884 A (LILLY CO ELI) 14 August 2020 (2020-08-14) * the whole document * ----- | 1-15 | TECHNICAL FIELDS SEARCHED (IPC) G16H |

The present search report has been drawn up for all claims

| Place of search | Date of completion of the search | Examiner |
|---|---|---|
| Munich | 10 June 2022 | Laub, Christoph |

EPO FORM 1503 03.82 (P04C01)

# EP 4 207 210 A1

## ANNEX TO THE EUROPEAN SEARCH REPORT
## ON EUROPEAN PATENT APPLICATION NO.

EP 21 21 8042

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2022

| Patent document cited in search report | | | Publication date | Patent family member(s) | | | Publication date |
|---|---|---|---|---|---|---|---|
| CA 3055770 | A1 | | 27-09-2018 | CA | 3055770 | A1 | 27-09-2018 |
| | | | | CN | 110650683 | A | 03-01-2020 |
| | | | | EP | 3600036 | A1 | 05-02-2020 |
| | | | | US | 2018271455 | A1 | 27-09-2018 |
| | | | | US | 2018272063 | A1 | 27-09-2018 |
| | | | | US | 2018272064 | A1 | 27-09-2018 |
| | | | | US | 2018272065 | A1 | 27-09-2018 |
| | | | | US | 2018272066 | A1 | 27-09-2018 |
| | | | | US | 2018277242 | A1 | 27-09-2018 |
| | | | | US | 2018277246 | A1 | 27-09-2018 |
| | | | | US | 2021259641 | A1 | 26-08-2021 |
| | | | | US | 2022096021 | A1 | 31-03-2022 |
| | | | | WO | 2018175935 | A1 | 27-09-2018 |
| CN 101689224 | A | | 31-03-2010 | CA | 2687562 | A1 | 31-12-2008 |
| | | | | CN | 101689224 | A | 31-03-2010 |
| | | | | DK | 2562664 | T3 | 01-02-2021 |
| | | | | EP | 2171630 | A1 | 07-04-2010 |
| | | | | EP | 2562664 | A2 | 27-02-2013 |
| | | | | ES | 2845400 | T3 | 26-07-2021 |
| | | | | HK | 1142421 | A1 | 03-12-2010 |
| | | | | JP | 5209714 | B2 | 12-06-2013 |
| | | | | JP | 5529307 | B2 | 25-06-2014 |
| | | | | JP | 2010532044 | A | 30-09-2010 |
| | | | | JP | 2013122790 | A | 20-06-2013 |
| | | | | KR | 20100027165 | A | 10-03-2010 |
| | | | | KR | 20130010037 | A | 24-01-2013 |
| | | | | US | 2009006061 | A1 | 01-01-2009 |
| | | | | WO | 2009002620 | A1 | 31-12-2008 |
| US 2020282141 | A1 | | 10-09-2020 | CA | 3071570 | A1 | 07-02-2019 |
| | | | | EP | 3438858 | A1 | 06-02-2019 |
| | | | | JP | 2020529230 | A | 08-10-2020 |
| | | | | KR | 20200037233 | A | 08-04-2020 |
| | | | | US | 2020282141 | A1 | 10-09-2020 |
| | | | | WO | 2019025506 | A1 | 07-02-2019 |
| JP 2005326943 | A | | 24-11-2005 | JP | 4273036 | B2 | 03-06-2009 |
| | | | | JP | 2005326943 | A | 24-11-2005 |
| CN 111542884 | A | | 14-08-2020 | AU | 2018388898 | A1 | 11-06-2020 |
| | | | | AU | 2022201374 | A1 | 24-03-2022 |
| | | | | CA | 3085930 | A1 | 27-06-2019 |
| | | | | CN | 111542884 | A | 14-08-2020 |
| | | | | EP | 3729446 | A1 | 28-10-2020 |
| | | | | JP | 2021507395 | A | 22-02-2021 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 21 21 8042

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2022

| Patent document cited in search report | Publication date | Patent family member(s) | Publication date |
|---|---|---|---|
| | | US 2020342974 A1 | 29-10-2020 |
| | | WO 2019125932 A1 | 27-06-2019 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GHOSHAL, BIRAJA ; TUCKER, ALLAN ; SANGHERA, BAL et al.** Estimating uncertainty in deep learning for reporting confidence to clinicians in medical image segmentation and diseases detection. *Computational Intelligence,* 2021, vol. 37 (2), 701-734
- **PAPERNOT, NICOLAS ; MCDANIEL, PATRICK.** Deep k-nearest neighbors: Towards confident, interpretable and robust deep learning. *arXiv preprint arXiv,* 2018
- **ROMAN HOVORKA et al.** Nonlinear model predictive control of glucose concentration in subjects with type 1 diabetes. *Physiol Meas.,* 2004, vol. 25, 905-920
- **ROMAN HOVORKA et al.** Partitioning glucose distribution/transport, disposal, and endogenous production during IVGTT. *Physical Endocrinol Metab,* 2002, vol. 282, E992-E1007